# EUROPEAN PATENT APPLICATION

(11) **EP 2 979 652 A1**
(43) Date of publication of application: **03.02.2016**
(21) Application number: 14178707.7
(22) Date of filing: 28.07.2014
(51) Int. Cl.: A61B 17/70

(54) **Surgical instruments**

(71) Applicant: Universitat de Barcelona, 08028 Barcelona (ES)
(72) Inventor: Prats Galino, Alberto, 08028 Barcelona (ES); Pereira Carpio, David Othoniel, 08036 Barcelona (ES)

(57) **Abstract**

Methods for laminoplasty are disclosed, as well as surgical instruments and surgical implants related to these methods.

## Description

The present disclosure relates to surgical interventions accessing the spinal canal, and in particular to methods of treating spinal stenosis. The present disclosure further relates to implants, surgical instruments and surgical kits used in such methods. The present disclosure further relates to scalpels, and in particular to scalpels for use in minimally invasive procedures.

### BACKGROUND ART

Spinal stenosis is an abnormal narrowing (stenosis) of the spinal canal that can occur in regions of the spine. This narrowing causes a restriction of the spinal canal, resulting in a neurological deficit. Symptoms can include pain, numbness, and loss of motor control.

There are several types of spinal stenosis, with lumbar stenosis and cervical stenosis being the most frequent. Spinal stenosis more frequently occurs in the elderly.

One possible treatment includes surgery. The traditional technique for decompression of lumbar nerves is laminectomy, which is a surgical procedure in which a lamina and spinous process are permanently removed to gain access to the spinal canal and its contents or to decompress it.

Laminectomy may be an effective procedure for relieving pressure on spinal nerves, but may result in chronic back pain and weakness in a patient after the laminectomy. This is generally considered to be caused by the destruction of the lamina, spinous process, and interspinous and supraspinous ligaments.

An alternative technique is laminoplasty. Different types of laminoplasty are known including e.g. the "open door" technique and "double door" technique. More so than in traditional laminectomy, the lamina, spinous process, inter-and supraspinous ligaments are substantially preserved in laminopasty.

In an "open door" technique, a selected lamina undergoes a laminotomy, whereas in a contralateral lamina, a hinge is created. Domes of the spinous process of the affected vertebrae may be removed.

Cutting the lamina and separating it from the articular process is a delicate process in which it is of the utmost importance not to damage underlying tissues. During this process a surgeon may generally use a drill and a ronguer in the process of cutting through the lamina.

Subsequently, a portion of the yellow ligament may be cut in order to open the spinal canal. A surgeon may generally use a blunt dissector to separate tissues and use a separate scalpel to cut the ligament in a step-by-step process, i.e. tissues are separated over a small stretch, then along that stretch the lamina can be cut, then again tissue is separated over a following stretch, and again a cut is made, and so no. A surgeon thus has to frequently change instruments, making the operation cumbersome and less precise.

After cutting the lamina on either side and after cutting the yellow ligament, the lamina can then be folded open, while still being connected to articular process using the laminar hinge. Opening of the lamina creates sufficient space in the spinal canal and thus resolves the spinal stenosis. In order to stabilize a lamina, implants can be used which extend between the lamina and its articular process. Such implants generally consist of a plate which comprises several laminar holes and several holes for articular process screws. Several bone screws may be used to attach the implant to the lamina and to the articular process.

One major disadvantage linked to the "open door" technique is the relatively risky procedure of attaching the implant to the relatively vulnerable and thin lamina. Many surgeons are afraid to use the open door technique because they are afraid to damage the spinal cord.

In a double door technique on the other hand, the spinous process is split along the midline. A surgical implant ("spacer") may be positioned between the two halves of spinous process to keep the spinal channel open and at the same time protect the spinal cord. This technique however is also very challenging even for very well trained professionals.

There thus exists a need for new methods for resolving spinal stenosis, as well as surgical tools and implants especially adapted for these methods.

### SUMMARY

In a first aspect, a method for the surgical treatment of spinal stenosis is provided wherein a spinous process of an affected vertebra is shortened by removing a mid portion of the spinous process, severing the right and left laminae of the vertebra from the articular process, attaching the laminae to the tip of the spinous process, providing a first implant between the right lamina and the right articular process and providing a second implant between the left lamina and the left articular process and attaching the laminae and articular processes to the two implants.

In accordance with this aspect, a method is provided which resolves at least some of the aforementioned problems. One important aspect is that the tip of the spinous process of the vertebra remains aligned with neighboring vertebrae. After the surgical intervention, the spine may thus be more suited for withstanding mechanical loads in comparison to prior art interventions.

In a second aspect, a surgical implant device for stabilising a lamina of a vertebra of a patient is provided. The device comprises a mounting flange including a hole for accommodating a bone screw for attachment to articular process of the vertebra, and a first, a second and a third clamping flange. The first clamping flange is configured to be arranged on a first side of the lamina, and the second and third clamping flanges are configured to be arranged on a second side of the lamina, the second side being opposite to the first side, such that the lamina is clamped between the clamping flanges. The clamping flanges comprise one or more sharp stabilizers on surfaces that are in contact with the lamina and the second and third clamping flanges are laterally offset with respect to the first clamping flange.

In accordance with this aspect, an implant is provided which is particularly suited for the surgical intervention described above. Clamping flanges are provided on either side of the lamina and due to the sharp stabilizers on the clamping flanges, no bone screws are necessary for fixing the implant to the laminae. The clamping flanges on one side can be laterally offset with respect to the clamping flange on the other side. This ensures a stable clamping of the lamina, and stable positioning of the lamina in spite of having only a single screw hole for attachment to the articular process. Risky procedures for attaching the implant to the laminae may thus be avoided, while stability of the laminae is maintained.

In some examples, the inner clamping flange and/or the outer clamping flange are flexible such that their orientation with respect to the mounting flange is adjustable for fitting the clamping flanges on either side of the lamina. The flexibility of the clamping flanges enables relatively easy positioning of the flanges on either side of a lamina.

In some examples, two outer clamping flanges and a single inner clamping flange is provided. Alternatively, two inner clamping flanges and a single outer clamping flange is provided. In these examples, stability of the lamina can be further improved.

In another aspect, a surgical kit is provided. Such a kit may comprise a surgical implant device substantially as hereinbefore described, and one or more bone screws for attachment of the implant to the articular process of the vertebra. Optionally, the surgical kit may further include a scalpel and/or an osteotome.

In another aspect, a scalpel for minimally invasive surgical procedures is provided. The scalpel comprises a longitudinal shaft having a proximal end and a distal end, with a grip being provided at the proximal end, and a dissector being provided at the distal end. The dissector comprises a first portion extending along a first direction generally corresponding to a direction of the distal end of the shaft, and a second portion extending along a second direction, wherein an angle between the second direction and the first direction is 90° - 135°. The distal end of the second portion comprises a rounded tip. The dissector further comprises a cutting blade provided between the first portion and the second portion of the dissector, wherein a cutting edge of the cutting blade is arranged forming an acute angle with the first direction such that the scalpel can dissect and cut tissue in a single substantially straight movement.

In accordance with this aspect, a scalpel is provided which can at the same time separate tissue and cut tissue. Moreover this can be done in a single movement, because the cutting edge has at least a component along the first direction, i.e. is not perpendicular to the first direction. Such a scalpel is particularly suited for use during a surgical procedure substantially as herein before described. However such a scalpel may also be useful in other surgical interventions.

In some examples, the dissector may be releasably coupled to the distal end of the longitudinal shaft. In such a case, the dissector may be made for single use, i.e. disposable, whereas the shaft including a handle or grip may be made for multiple uses. Problems related to degradation of the cutting blade and/or problems related to repeated sterilization may thus be avoided. In an alternative example, the entire scalpel is for single use only, i.e. disposable.

In some examples, the longitudinal shaft may comprise a first shaft portion extending from the proximal end to a transition, and a second shaft portion extending from the transition to the distal end, and wherein the first and second shaft portions are substantially parallel, and wherein the transition is not parallel to the first and second shaft portions. According to this examples, the longitudinal shaft with grip for the surgeon is displaced with respect to the portion of the device entering the patient's body. This may improve visualization by the surgeon during procedures.

In preferred examples, the distal end of the longitudinal shaft is of reduced cross-section as compared to the proximal end of the longitudinal shaft. In these examples, the shaft with grip for holding by the surgeon can be more easily held and managed, whereas the portions of the scalpel which in use are inserted into a patient's body can be of small dimensions.

In a further aspect, an osteotome is provided. The osteotome comprises a first jaw and a second jaw, and a first grip connected to the first jaw and a second grip connected to the second jaw. The first and second jaws are pivotable around a first axis and facing each other. The first jaw and the second jaw comprise substantially parallel first and second cutting members, wherein the first cutting members of the first and second jaw are arranged symmetrically with respect to a central plane, and the second cutting members of the first and second jaw are arranged symmetrically with respect to the central plane. The osteotome can ensure a constant distance between a first and a second cutting plane on the first jaw and on the second jaw. No separate cuts are necessary to cut spinous process. The portion of spinous process that is separated from the rest of the spinous process may thus have a constant width. Also because the cutting planes are parallel, it can be ensured that the resulting apical portion of the spinous process will fit with the resulting base of the spinous process.

In some examples, the first and second cutting members are arranged parallel to each other at a distance corresponding to approximately one third of a spinous process of a vertebra. As such, the osteotome can be adapted specifically for the methods substantially as herein described.

In some examples, the cutting members may be arranged on internal surfaces of the first and second jaws, the cutting members may be substantially V-shaped. Four cutting planes may thus be defined (two on the first jaw and two on the second jaw). This may make fitting the apical portion and base portion of the spinous process easier.

### BRIEF DESCRIPTION OF THE DRAWINGS

Non-limiting examples of the present disclosure will be described in the following, with reference to the appended drawings, in which:
Figures 1a - 1c schematically illustrate an example of a method of surgical treatment of spinal stenosis;
Figures 2a - 2d schematically illustrate an example of an implant device in accordance with an implementation;
Figures 3a - 3c schematically illustrate the implant device of figure 2 attached to a vertebra in accordance with an implementation;
Figures 4a - 4c schematically illustrate an example of a scalpel;
Figures 5a and 5b illustrate further examples of a scalpel;
Figures 6a - 6d illustrate an example of an osteotomes according to an implementation; and
Figure 6e schematically illustrates a further example of an osteotome.

### DETAILED DESCRIPTION OF EXAMPLES

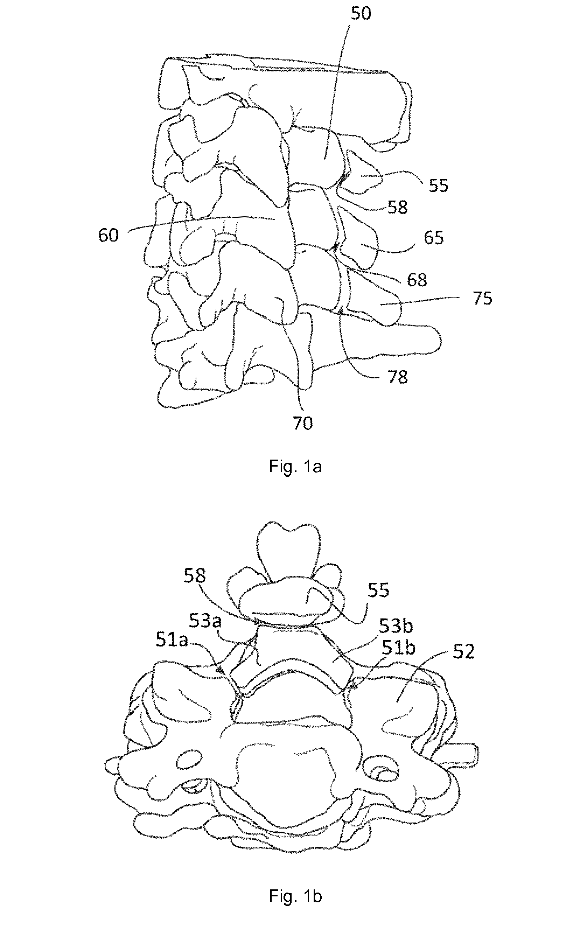

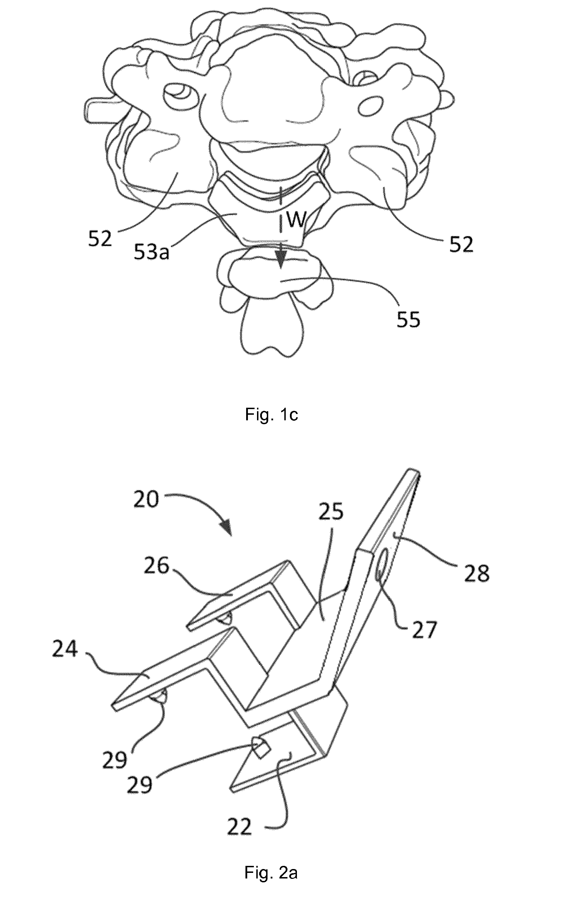

Figure 1 illustrates various steps of an example of a method of surgical treatment of spinal stenosis. Figure 1a illustrates three cervical vertebrae 50, 60, 70. In a first method step the "domes" 55, 65, 75 or apical portions of the spinous process of vertebrae 50, 60, 70 are separated by removing a mid-portion of the spinous process.

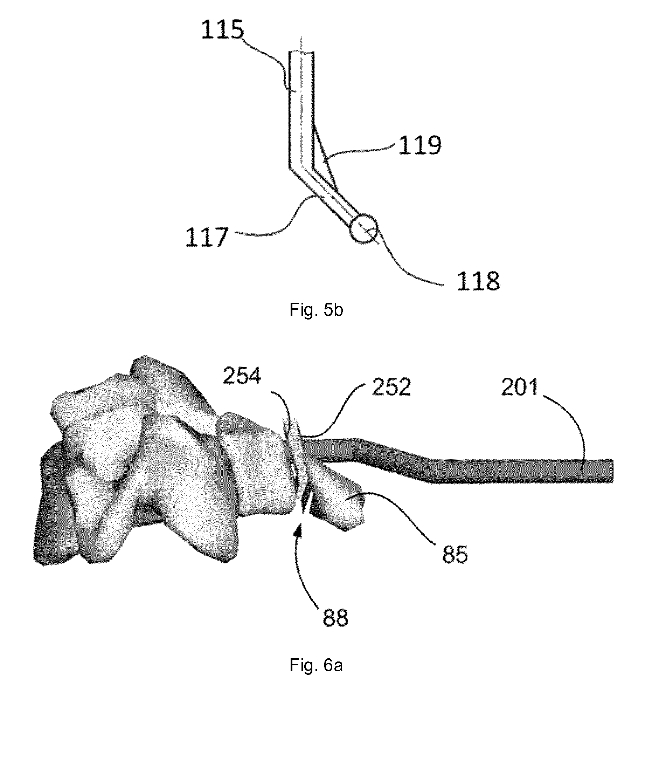

Removal of the mid-portion of the spinous process may be done using an osteotome adapted to cut in two planes simultaneously. Examples of such osteotomes are described later herein, particularly with reference to figures 6a - 6e.

In an example, approximately one third of the spinous process may be left attached to the laminae, approximately one third may be removed and approximately one third of the spinous process may form the apical portion.

In figure 1a, the separation of the apical portions 55, 65, and 75 from the base of the spinous process and the laminae is schematically illustrated with cuts 58, 68 and 78.

In a subsequent step (figure 1 a, right), lamina 53a and 53b may be separated using cuts 51 a and 51 b from articular process 52 of vertebra 50. Similar steps may be performed on vertebrae 60 and 70.

Figure 1b schematically illustrates a top view of the vertebrae after laminotomy.

In a further non-illustrated step, yellow ligament may be cut above vertebra 50 and below vertebra 70 and along longitudinal lines there between. The spinal canal may then be effectively opened and e.g. nerves may be decompresses. In another example, a similar method could be carried out to gain access e.g. to a tumor in the spinal canal.

Figure 1c shows a top view of the vertebrae in which the lamina (with a portion of the spinous process) can be moved along direction W to be attached to the previously separated apical portions. These portions of spinous process may be sutured together. The result is that the apical portions ("domes") of the spinous process of treated vertebrae after the surgical intervention remain aligned with the domes of untreated vertebrae.

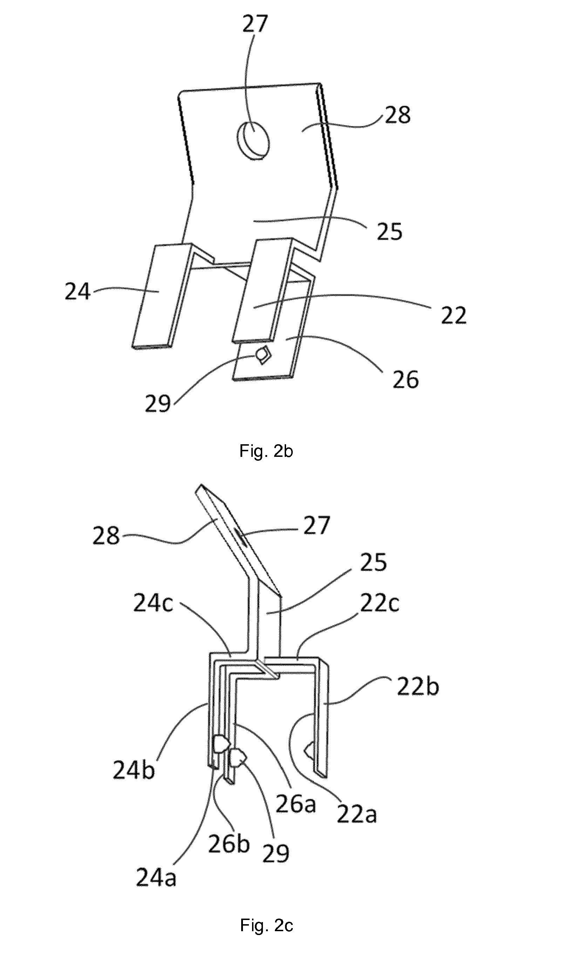

In order to stabilise the laminae, an implant device 20 such as illustrated in figures 2a - 2d may be used. The implant device 20 may comprise an inner clamping flange 22, and two outer clamping flanges 24 and 26. The inner clamping flange is adapted to be arranged on an internal side of the lamina (i.e. the side of the lamina facing the spinal channel) and the outer clamping flanges are adapted to be fitted on an external side of the lamina (i.e. the side of the lamina facing away from the spinal channel).

The inner clamping flange 22 comprises a laminar face 22a, and a spinal face 22b. Outer clamping flanges 24 and 26 comprise laminar faces 24a and 26a and external faces 24b and 26b. The laminar faces 22a, 24a, and 26a may comprise one or more spikes 29 or e.g. sharp pins, which serve as sharp stabilizing elements. In use, laminae may be clamped between the clamping flanges 22 on the one hand and outer clamping flanges 24 and 26 on the other hand. In operation, the spikes may protrude into the lamina to securely fix the lamina with respect to the implant device.

The implant device may furthermore comprise a mounting flange 28 having at least one hole 27 for accommodating a fastener, e.g. a bone screw. The implant device may thus be securely fastened to the lateral process of a vertebra.

As may be seen in figures 2a and 2b, the flanges 24 and 26 are laterally offset with respect to flange 22. That is, the flanges 24 and 26 do not overlap or only partially overlap flange 22. The lateral offset between the flanges improve the stability of the implant and lamina. In spite of having a single fixation point with a bone screw through hole 27 at the articular process, the stability of the laminae can be guaranteed.

A flat spacer 25 may extend between mounting flange 28 and clamping flanges 22, 24 and 26. The flat spacer 25 may be arranged to form an obtuse angle with respect to the mounting flange. The obtuse angle may be e.g. approximately 135°.

The clamping flanges 22, 24 and 26 may be substantially L-shaped. Each of the flanges may include a first portion 22c, 24c, 26c which is substantially perpendicular to the spacer 25, and a second portion which is substantially parallel to the spacer 25.

In the illustrated example, two outer clamping flanges and a single inner clamping flange was provided. Although this is generally a preferred arrangement, in an alternative example, two inner clamping flanges and a single outer clamping flange are provided.

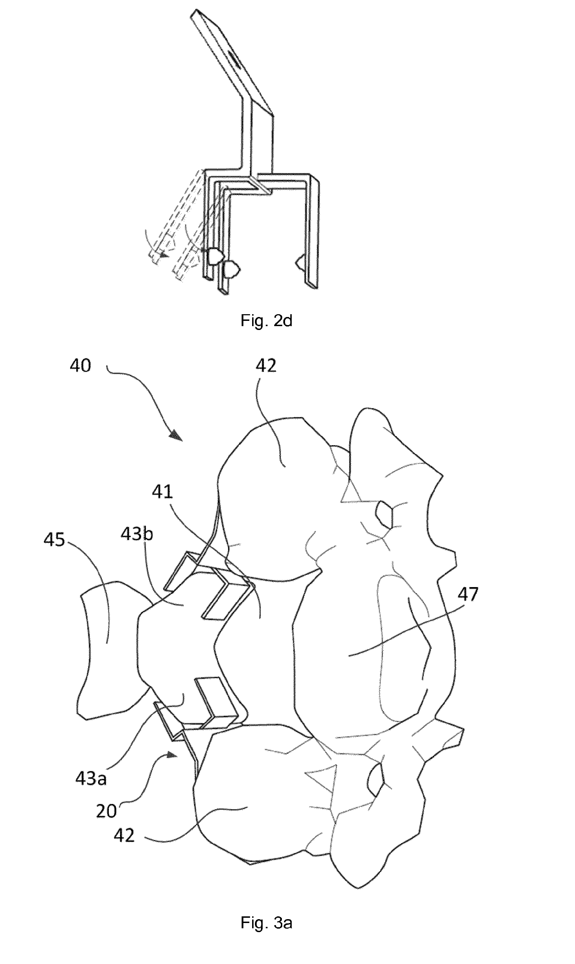

Figure 2d illustrates that the clamping flanges on one side of the lamina (or on both sides of the lamina) may be relatively flexible. One or more of the clamping flanges can be deformed after introduction into a patient's body. A surgeon can, using his hands or a dedicated tool, bring the clamping flanges on either side of the lamina closer to each other and in contact with the lamina. The spikes or sharp pins on the clamping flanges can protrude into the lamina and fix the implant on the lamina. As a result, the clamping flanges on either side of the lamina may be substantially parallel to each other.

Before introduction into a patient's body, the clamping flanges on the inside and outside of the lamina are not necessarily parallel to each other.

It is generally easier and less risky to fold the clamping flanges arranged on an external side of the lamina, i.e. facing away from the spinal canal than on an internal side of the lamina. For these reasons, preferably two clamping flanges are provided externally, and a single clamping flange is provided internally.

Figures 3a - 3c schematically illustrate the implant device 20 in use in a surgical method e.g. for treatment of spinal stenosis. Reference sign 41 indicates the spinal canal which may get restricted requiring a surgical intervention. A vertebra 40 is shown comprising articular process 42, body 47, laminae 43a and 43b and the apical portion of the spinous process 45. In a previous step, a mid-portion of the spinous process has been removed.

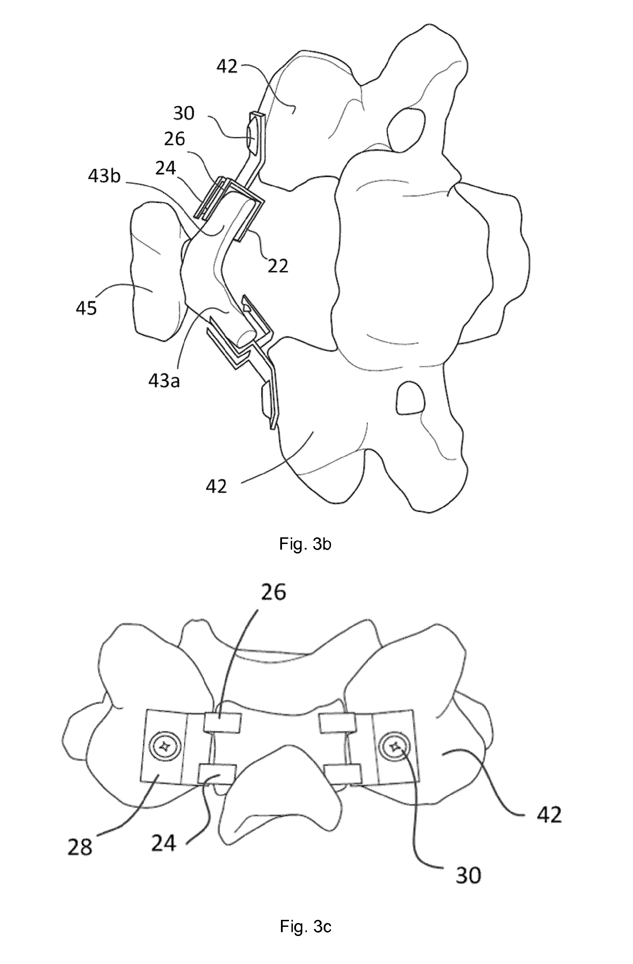

Figures 3b and 3c illustrate, that mounting flanges 28 may be attached to the articular process using bone screws 30. The clamping flanges may be seen as stabilizing and fixing laminae 43a and 43b in their position. As previously described, in order to position an implant 20, the clamping flanges may have a certain flexibility such that they can be fitted more easily on either side of the laminae and subsequently pinched onto and fixed onto the laminea.

In the thin laminae, bone screws can be avoided, because spikes on the clamping flanges clamp down on the lamina. Bone screws can be used on the articular process, where the risk of damaging tissues or nerves while screwing is lower than in the case of the laminae.

Even though the previous examples were explained with reference in particular to cervical vertebrae, it will be clear that similar methods and devices may be used for surgical interventions involving other vertebrae as well, e.g. thoracic or lumbar vertebrae. The size of the implant devices could be selected for a specific vertebra and for a specific patient (group).

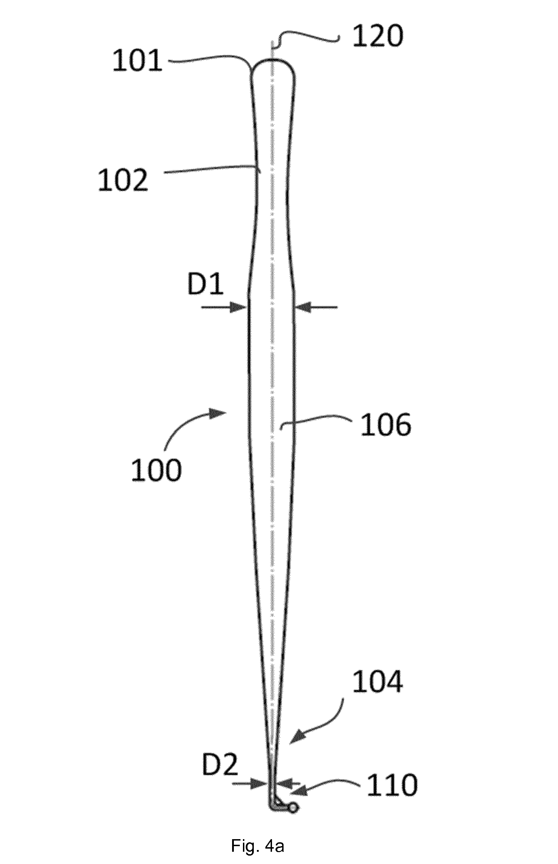

Figures 4a - 4c schematically illustrate an example of a surgical scalpel in accordance with an implementation. The scalpel 100 may comprise a longitudinal shaft 106 extending generally along an axis 120. A proximal portion 101 of the longitudinal shaft may comprise a handle or a gripping portion 102 such that a surgeon can easily hold and manipulate the scalpel. At a distal end 104 of the shaft, a dissector 110 may be attached or coupled to the shaft.

In this example, the gripping portion is slightly thinner than the other parts of the longitudinal shaft 106. In another example, the gripping portion may be of substantially the same thickness as the rest of the shaft 106. Optionally, roughness of corrugations may be provided to form a gripping portion.

The dissector may comprise a first portion 110 extending generally along longitudinal shaft 102 and a second portion 117. In this example, the second portion may be substantially perpendicular to the first portion 115.

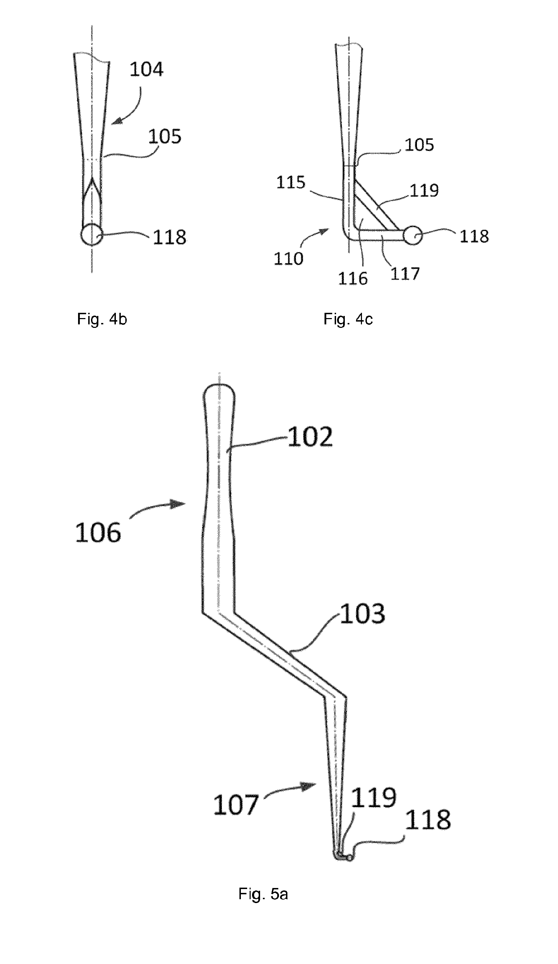

Between first portion 115 and second portion 117 a cutting blade 119 may be foreseen. In the shown example, the cutting blade is arranged at a 45° angle with respect to both the first and second portion. The cutting edge thus extends at least partially in a direction to the right in figures 4a and 4c, i.e. in a direction towards a tip 118 of the second portion. The tip 118 may be substantially rounded such as not to damage or cut tissue, but rather to separate tissue layers from each other. The cutting blade may be supported by a substantially triangular portion 116 extending between first portion 115 and second portion 117. The triangular portion may be integrally formed with the first portion 115 and second portion 117.

In this example, the tip 118 is shown to be substantially ball-shaped, but it will be clear that other rounded shapes can serve the same purpose. Since the cutting edge is arranged at least facing the tip to some extent, as the scalpel is moved forward, i.e. to the right (in the case of figures 4a and 4c), the scalpel can safely separate tissue layers and cut the appropriate tissue at the same time, in a single movement.

In an example, a diameter of the rounded tip 118 may be approximately 2 mm. The diameter of a circular cross-section of first portion 115 and second portion 117 may be e.g. approximately 1.5 mm. The length of the portions may be e.g. approximately 7.5 mm.

Such a scalpel may be advantageous in several different surgical procedures, including e.g. a surgical method substantially as herein before described. With prior art scalpels and dissectors, when cutting the yellow ligament continuous switching between a blunt dissector and a cutting instrument was necessary. With a scalpel according to the present disclosure, this can be avoided. With a single instrument both separation of the tissue and cutting of the ligament can be achieved.

The longitudinal shaft may have a generally circular cross-section. At a distal end, a diameter D1 of the shaft may be between 4 and 15 mm. The length of the shaft from the proximal end to the end of the dissector portion may be e.g. about 20 cm. The diameter D2 at a distal end of the shaft 106 may be generally reduced as compared to the diameter D1 and may be e.g. 1.5 mm. A ratio of diameter D1 to diameter D2 may be e.g. 3 - 10. A strong reduction in cross-section from the proximal end to the distal end allows a good grip for a surgeon at a thicker portion of the scalpel, and at the same time allows the scalpel to be used in minimally invasive surgeries wherein the opening into a patient's body during surgery may be very small.

In an example, the dissector portion 110 may be releasably coupled at a distal end of the shaft 106. The dissector portion may be disposable and thus thrown away after a surgical procedure. The shaft 106 with a gripping portion could be sterilized and re-used in further procedures, wherein another disposable dissector portion is coupled again at its distal end.

In an alternative example, the whole scalpel may be disposable. The dissector portion may completely or partially in some examples be integrally formed with the first shaft.

Figures 5a and 5b schematically show two further alternative examples of scalpels according to further implementations. The example of figure 5a is generally comparable to the scalpel illustrated in figure 4, but the scalpel may comprise a first shaft portion 106, and a second shaft portion 107 which extend generally along lines that are parallel to each other. In between the first shaft portion 106 and the second shaft portion 107, a transition portion 103 is provided. This arrangement ensures that the proximal end portion, with grip 102, is offset with respect to a distal end portion of second shaft 107. This allows a surgeon to hold the scalpel at one end, and maintain improved visibility of the opening in the body of the patient in general and of the distal end of the scalpel in particular. Better visibility during dissecting and cutting may thus be achieved. As in the previous example, a dissector portion including a rounded tip 118 and a cutting blade 119 is provided.

Figure 5b shows a further example, which may be combined with the longitudinal shafts of any of the scalp examples shown in figures 5a and figures 4a - 4c. The dissector portion of the scalpel in this example may comprise a first portion 115 extending generally along a direction of the distal end of the shaft, and a second portion 117 forming an obtuse angle with the first portion. In the particular example shown, the angle between the first and second portion may be approximately 135°. A cutting blade extends between the first and second portions 115 and 117 respectively. As before, a cutting edge faces at least in a direction towards the rounded tip 118, which allows separating tissues and cutting tissue simultaneously.

In an example, the angle between the first portion 115 and the cutting edge 119 may be between 15° and 30°.

Figures 6a - 6d schematically illustrate a first example of an osteotome that may be of particular use in previously explained surgical methods. As previously explained, a mid portion of the spinous process is removed in order to separate an apical portion of the spinous process from the laminae (and attached spinous process). The removal of the mid portion of the spinous process allows the laminae to be moved towards the apical portion of the spinous process (i.e. it allows opening or widening of the spinal canal), while at the same time, the apical portions of the spinous process stay in place. Mechanically, the resulting structure of the vertebrae in comparison with respect to prior art techniques may have significant advantages.

In preferred methods, the mid portion of the spinous process that is removed is substantially v-shaped. Such a v-shape enables easier fitting and subsequent attachment of the remaining spinous process portions.

Figure 6a very schematically illustrates one half of an example of an osteotome used to create a cut 88, to remove a mid portion of spinous process and thereby separate an apical portion 85 from a base portion of the spinous process. For ease of understanding, a jaw portion supporting the cutting members is not shown in this figure.

In this example, two substantially parallel V-shaped cuts are made using cutting members 252 and 254. With a single movement of the osteotome, the two cuts can be made, from either side of a spinous process.

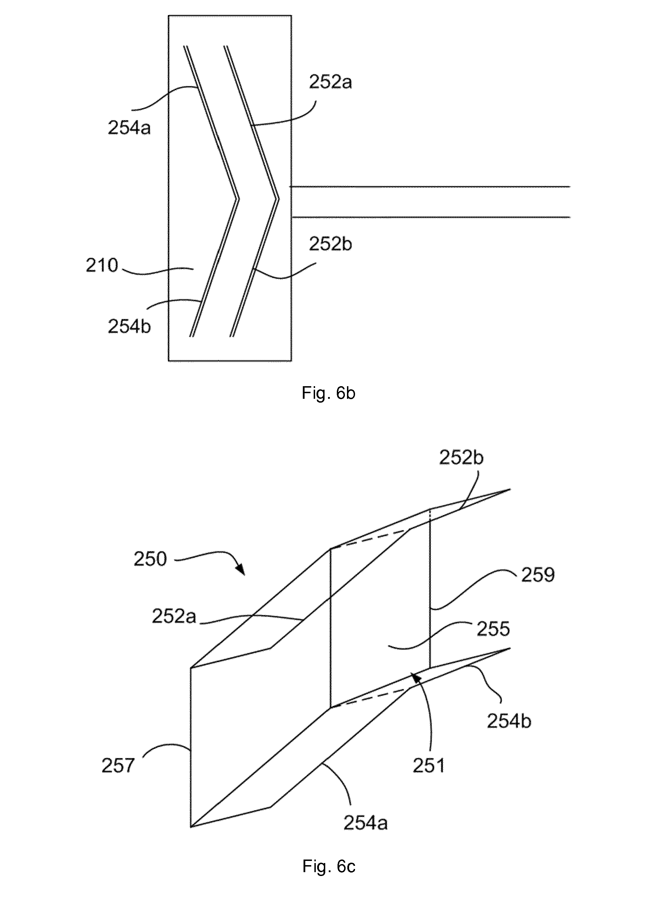

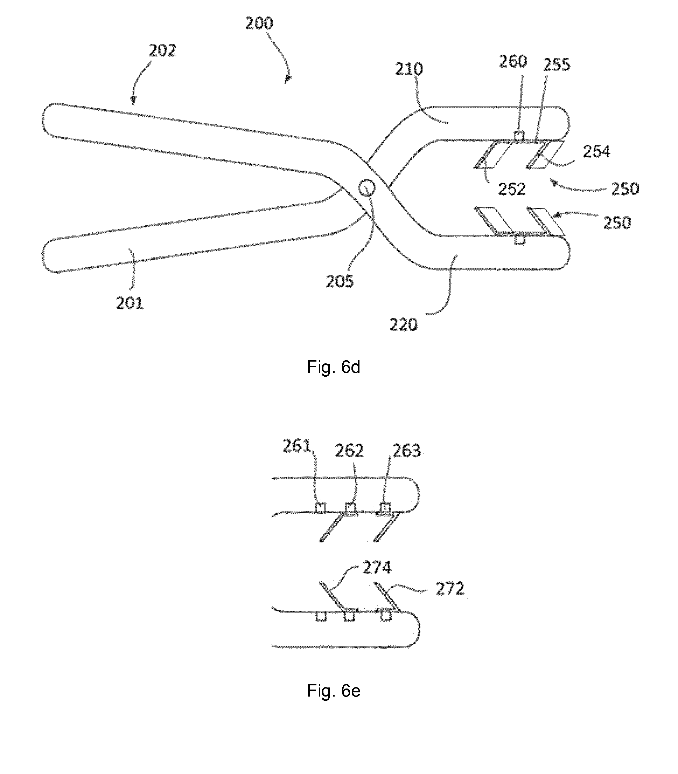

Figure 6b schematically illustrates a view inside a jaw 210 of the osteotome previously illustrated .Figure 6c schematically illustrates a cutting blade 250 comprising both first and second cutting member 252 and 254. And figure 6d illustrates a cross-sectional view along a central plane of the osteotome.

The osteotome 200 of this example may comprise a first jaw 210 and a second jaw 220 generally facing each other. A first grip 201 may be operationally connected with the first jaw 210 and a second grip 202 may be operationally connected with the second jaw 220.

First jaw 210 and second jaw 220 define a cutting area between them. The jaws may pivot about axis to bring the jaws closer to each or further away from each other.

The first jaw 210 may comprise substantially parallel cutting members 252 and 254. Similar cutting members are provided on the second jaw. The cutting members of the first and second jaws are generally arranged symmetrically with respect to a middle plane. Using the grips 201 and 202, the jaws 210 and 220 may be brought closer together. Parallel cutting planes are thus formed between the first and second cutting members 252 and 254, both on the first and second jaw.

First cutting member 252 and second cutting member 254 may be substantially v-shaped. In preferred examples, an internal angle between the legs 252a and 252b and 254a and 254b of the V may be between 90° and 160°, preferably between 120° and 150.

The distance between the cutting members defines the size of the portion of spinous process that is removed. The parallel cutting planes ensure that the portion of the spinous process that is removed is constant with every use of the osteotome. If a normal osteotome were used, two separate cuts would have to be made and it would be very hard or nearly impossible to ensure a constant distance between the cuts.

Since the size of the portion of spinous process that is removed is constant, the distance that the laminae can be moved is constant as well, and the size of the implant can be constant as well. The distance between the cutting members may correspond to approximately one third of the spinous process. In the particular example of figures 6a - 6d, a single cutting blade 250 is provided both on the first and the second jaw. The cutting members 252 and 254 may thus be integrally formed.

The cutting blade 250 comprises parallel cutting members 252 and 254 connected by flat potion 251. The flat portion 251 comprises a fastener which can engage with an attachment member 260 on the jaws. For example, the fastener may be e.g. a protrusion extending into the attachment member 260. The fastener may comprise a screw thread in some implementations. In alternative implementations, the attachment and fastener may have a hexagonal cross-section wherein the fastener is fitted into the attachment member 260.

The cutting blade 250 may extend between edges 257 and 259, which defines a length of the cutting planes. In the example of figure 6b, the side edges 257 and 259 are shown to be open. In an alternative example, the side edges 257 and 259 may be closed by side walls.

Figure 6e shows a further example of an osteotome. In this example, both the first jaw and the second jaw comprise a plurality of attachment points 261, 262 and 263. The cutting members 272 and 274 are not integrally formed, but instead are different components. The attachment members 261, 262 and 263 may be generally similar so that, at the election of the surgeon, the cutting members may be attached at different points to adjust the distance between cutting members. The distance may be adjusted e.g. from 3 mm to 5 mm or 7 mm.

In the illustrated example, the jaws 210 and 220 may be brought together to cut a bone manually, i.e. by the surgeon's hands. In an alternative example, an automated manner of cutting may be provided. In one example, a screw may connected grips 201 and 202 and by adjustment of the screw (in an automated manner), the jaws 210 and 220 can pivot about axis 205.

Also, although in the illustrated examples, the jaw supporting the cutting members was shown to be substantially straight, in alternative examples, the jaws may be more arched.

Various embodiments of the present disclosure are set out in the following numbered clauses:
Clause 1. A scalpel for minimally invasive surgical procedures comprising
   - a longitudinal shaft having a proximal end and a distal end
   - a grip being provided at the proximal end, and
   - a dissector being provided at the distal end, wherein
   the dissector comprises a first portion extending along a first direction generally corresponding to a direction of the distal end of the shaft, and a second portion extending along a second direction, wherein an angle between the second direction and the first direction is 90° - 135°, wherein
   the distal end of the second portion comprises a rounded tip, and the dissector further comprising
   a cutting blade provided between the first portion and the second portion of the dissector, wherein
   a cutting edge of the cutting blade is arranged to generally face towards the rounded tip and/or to form an acute angle with the first direction such that the scalpel can dissect and cut tissue in a single substantially straight movement.
Clause 2. A scalpel according to clause 1, wherein the dissector is releasably coupled to the distal end of the longitudinal shaft.
Clause 3. A scalpel according to clause 2, wherein the dissector is disposable.
Clause 4. A scalpel according to clause 1, wherein the dissector is integrally formed with the longitudinal shaft.
Clause 5. A scalpel according to any of clauses 1 - 4, wherein the scalpel is disposable.
Clause 6. A scalpel according to any of clauses 1 - 5, wherein the second portion of the dissector is generally perpendicular to the first portion of the dissector.
Clause 7. A scalpel according to any of clauses 1 - 6, wherein a cutting edge of the cutting blade extends along a 45° angle to the first and second portions of the dissector.
Clause 8. A scalpel according to any of clauses 1 - 7, wherein the longitudinal shaft comprises a first shaft portion extending from the proximal end to a transition, and a second shaft portion extending from the transition to the distal end, and wherein the first and second shaft portions are substantially parallel, and wherein the transition is not parallel to the first and second shaft portions.
Clause 9. A scalpel according to any of clauses 1 - 8, wherein the distal end of the longitudinal shaft is of reduced cross-section as compared to the proximal end of the longitudinal shaft.
Clause 10. A scalpel according to clause 9, wherein the longitudinal shaft has a generally circular cross-section, and wherein a ratio between a diameter of the cross-section at the proximal end and a diameter of the cross-section at the distal end is 3 - 10.
Clause 11. A surgical implant device for stabilising a lamina of a vertebra of a patient, the device comprising
   a mounting flange including a hole for accommodating a bone screw for attachment to a articular process of the vertebra, and
   a first, a second and a third clamping flange,
   wherein the first clamping flange is configured to be arranged on a first side of the lamina, and the second and third clamping flanges are configured to be arranged on a second side of the lamina, the second side being opposite to the first side, such that the lamina is clamped between the clamping flanges,
   the clamping flanges comprising one or more sharp stabilizers on surfaces that are in contact with the lamina,
   wherein the second and third clamping flanges are laterally offset with respect to the first clamping flange.
Clause 12. A surgical implant according to clause 11, wherein the mounting flange, and clamping flanges are integrally formed.
Clause 13. A surgical implant according to clause 11 or 12, wherein one or more of the clamping flanges are flexible such that their orientation with respect to the mounting flange is adjustable for fitting the clamping flanges on either side of the lamina.
Clause 14. A surgical implant according to any of clauses 11 - 13, further comprising a substantially flat spacer between the mounting flange and clamping flanges.
Clause 15. A surgical implant according to clause 14, wherein the flat spacer is arranged at an obtuse angle with the mounting flange.
Clause 16. A surgical implant according to clauses 14 or 15, wherein one or more of the clamping flanges are flexible such that their orientation with respect to the spacer is adjustable for fitting the clamping flanges on either side of the lamina.
Clause 17. A surgical implant according to any of clauses 11 - 16, wherein the first clamping flange is configured to be arranged on an internal side of the lamina, and wherein the second and third clamping flanges are configured to be arranged on an external side of the lamina.
Clause 18. A surgical implant according to any of clauses 11 - 16, wherein the first clamping flange is configured to be arranged on an external side of the lamina, and wherein the second and third clamping flanges are configured to be arranged on an internal side of the lamina.
Clause 19. A surgical implant device according to any of clauses 11 - 18, wherein each of the outer clamping flange and inner clamping flanges comprises a single sharp stabilizer.
Clause 20. A surgical implant device according to any of clauses 11 - 19, wherein the sharp stabilizers are spikes.
Clause 21. A surgical implant according to any of clauses 11 - 20, wherein the clamping flanges are substantially L-shaped.
Clause 22. An osteotome comprising
   a first jaw and a second jaw, and
   a first grip connected to the first jaw and a second grip connected to the second jaw, wherein
   the first and second jaws being pivotable around a first axis and facing each other, and wherein
   the first jaw and the second jaw both comprise substantially parallel first and second cutting members, wherein optionally
   the first cutting members of the first and second jaw are arranged symmetrically with respect to a central plane, and
   the second cutting members of the first and second jaw are arranged symmetrically with respect to the central plane.
Clause 23. An osteotome according to clause 22, wherein the first and second cutting members are integrally formed.
Clause 24. An osteotome according to clause 22 or 23, wherein the first and second cutting members are arranged parallel to each other at a distance corresponding to approximately one third of a spinous process of a vertebra.
Clause 25. An osteotome according to any of clauses 22 - 24, wherein the first and second cutting members are separate components.
Clause 26. An osteotome according to any of clauses 22 - 25, wherein the first and second cutting members are substantially v-shaped.
Clause 27. An osteotome according to clause 26, wherein the legs of the v-shaped cutting members have an interior angle of 90 - 160°, preferably 120°-150°.
Clause 28. An osteotome according to any of clauses 22 - 27, comprising a plurality of attachment points for attachment of the cutting members.
Clause 29. A surgical kit comprising:
   - a surgical implant device according to any of clauses 11 - 21, and
   - a bone screw for attachment to the articular process of the vertebra.
Clause 30. A surgical kit according to clause 29, and further comprising a scalpel according to any of clauses 1 - 10.
Clause 31. A surgical kit according to clause 29 or 30, and further comprising an osteotome according to any of clauses 22 - 28.
Clause 32. A surgical kit according to any of clauses 28 - 30, wherein the surgical implant device is an implant device according to clause 13 and further comprising a tool for deforming the clamping flanges.
Clause 33. A surgical method comprising
   removing a mid portion of the spinous process,
   severing the right and left laminae of the vertebra from the articular process,
   attaching the laminae to an apical portion of the spinous process,
   providing a first implant between the right lamina and the right articular process and providing a second implant between the left lamina and the left articular process and
   attaching the laminae and articular processes to the two implants.
Clause 34. A method according to clause 33, wherein removing a mid portion of the spinous process comprises removing approximately one third of the spinous process.
Clause 35. A method according to clause 33 or clause 34, wherein removing a mid portion of the spinous process comprises cutting the spinous process with an osteotome according to any of clauses 22 - 28.
Clause 36. A method according to any of clauses 33 - 35, wherein attaching the laminae to the apical portion of the spinous process comprises attaching a base portion of the spinous process to the apical portion of the spinous process.
Clause 37. A method according to any of clauses 33 - 36, wherein attaching the laminae to the apical portion of the spinous process comprises suturing.
Clause 38. A method according to any of clauses 33 - 37, wherein the first and second implants are implants according to any of clauses 11 - 21. Clause 39. A method according to any of clauses 33 - 38, further comprising cutting yellow ligament.
Clause 40. A method according to clause 39, wherein the yellow ligament is cut using a scalpel according to any of clauses 1 - 10.
Clause 41. Use of a surgical method according to any of clauses 33 - 39 for the surgical treatment of spinal stenosis.
Clause 42. The use of a scalpel according to any of clauses 1- 10 for cutting yellow ligament.

Although only a number of examples have been disclosed herein, other alternatives, modifications, uses and/or equivalents thereof are possible. Furthermore, all possible combinations of the described examples are also covered. Thus, the scope of the present disclosure should not be limited by particular examples, but should be determined only by a fair reading of the claims that follow.

## Claims

1. A surgical implant device for stabilising a lamina of a vertebra of a patient, the device comprising
a mounting flange including a hole for accommodating a bone screw for attachment to a articular process of the vertebra, and
a first, a second and a third clamping flange,
wherein the first clamping flange is configured to be arranged on a first side of the lamina, and the second and third clamping flanges are configured to be arranged on a second side of the lamina, the second side being opposite to the first side, such that the lamina is clamped between the clamping flanges,
the clamping flanges comprising one or more sharp stabilizers on surfaces that are in contact with the lamina,
wherein the second and third clamping flanges are laterally offset with respect to the first clamping flange.

2. A surgical implant according to claim 1, wherein the mounting flange, and clamping flanges are integrally formed.

3. A surgical implant according to claim 1 or 2, wherein one or more of the clamping flanges are flexible such that their orientation with respect to the mounting flange is adjustable for fitting the clamping flanges on either side of the lamina.

4. A surgical implant according to any of claims 1 - 3, further comprising a substantially flat spacer between the mounting flange and clamping flanges.

5. A surgical implant according to claim 4, wherein the flat spacer is arranged at an obtuse angle with the mounting flange.

6. A surgical implant according to claim 4 or 5, wherein one or more of the clamping flanges are flexible such that their orientation with respect to the spacer is adjustable for fitting the clamping flanges on either side of the lamina.

7. A surgical implant according to any of claims -1 - 6, wherein the first clamping flange is configured to be arranged on an internal side of the lamina, and wherein the second and third clamping flanges are configured to be arranged on an external side of the lamina.

8. A surgical implant according to any of claims -1 - 6, wherein the first clamping flange is configured to be arranged on an external side of the lamina, and wherein the second and third clamping flanges are configured to be arranged on an internal side of the lamina.

9. A surgical implant device according to any of claims 1 - 8, wherein each of the outer clamping flange and inner clamping flanges comprises a single sharp stabilizer.

10. A surgical implant device according to any of claims 1 - 9, wherein the sharp stabilizers are spikes.

11. A surgical implant according to any of claims 1 - 10, wherein the clamping flanges are substantially L-shaped.

12. A surgical kit comprising:
- a surgical implant device according to any of claims 1 - 11, and
- a bone screw for attachment to the articular process of the vertebra.

13. A surgical kit according to claim 12, and further comprising a scalpel.

14. A surgical kit according to claim 12 or 13, and further comprising an osteotome.

15. A surgical kit according to any of claims 12 - 14, wherein the surgical implant device is an implant device according to claim 3 and further comprising a tool for deforming the clamping flanges.
